(19) Europäisches Patentamt / European Patent Office / Office européen des brevets

(11) **EP 1 755 742 B1**

(12) **FASCICULE DE BREVET EUROPEEN**

(45) Date de publication et mention
de la délivrance du brevet:
**22.06.2011 Bulletin 2011/25**

(51) Int Cl.:
***A61N 7/02*** *(2006.01)*

(21) Numéro de dépôt: 05743138.9

(22) Date de dépôt: **28.04.2005**

(86) Numéro de dépôt international:
**PCT/EP2005/051935**

(87) Numéro de publication internationale:
**WO 2005/107870 (17.11.2005 Gazette 2005/46)**

(54) **Dispositif de positionnement de moyens générateurs d'énergie d'un ensemble pour le traitement thermique de tissus biologiques**

Vorrichtung zur Positionierung der energieerzeugenden Mittel einer Konstruktion für die Wärmebehandlung biologischer Gewebe

Device for positioning the energy-generating means of an assembly for the heat treatment of biological tissues

(84) Etats contractants désignés:
**AT BE BG CH CY CZ DE DK EE ES FI FR GB GR HU IE IS IT LI LT LU MC NL PL PT RO SE SI SK TR**

(30) Priorité: **29.04.2004 FR 0404561**

(43) Date de publication de la demande:
**28.02.2007 Bulletin 2007/09**

(73) Titulaire: **Koninklijke Philips Electronics N.V.
5656 AE Eindhoven (NL)**

(72) Inventeurs:
• **MOONEN, Chretien
F-33170 Gradignan (FR)**
• **SALOMIR, Rares
F-69630 Chaponost (FR)**
• **MOUGENOT, Charles
F-33000 Bordeaux (FR)**

(74) Mandataire: **Damen, Daniel Martijn et al
Philips Intellectual
Property & Standards
High Tech Campus 44
5656 AE Eindhoven (NL)**

(56) Documents cités:
WO-A-03/059434    DE-C- 19 745 400
US-A- 5 443 068    US-A- 5 522 869
US-B1- 6 516 211    US-B1- 6 582 381

## Description

L'invention concerne le domaine du traitement de tissus biologiques par hyperthermie, et plus particulièrement le traitement de tissus mammaires.

Les thérapies par hyperthermie sont des techniques couramment utilisées pour traiter localement des tissus biologiques. Elles consistent à chauffer au moyen d'une source d'énergie (laser, micro-ondes, ondes radiofréquences, ultrasons) une zone cible du tissu biologique.

D'une manière générale, les thérapies par hyperthermie locale permettent des interventions médicales dont la nature invasive est réduite au minimum. Parmi les types d'énergie utilisés, les ultrasons focalisés (FUS) sont particulièrement intéressants puisqu'ils permettent de chauffer une zone cible, de manière non invasive et en profondeur dans les tissus.

Le document FR 2 823 678 (publié le 25 octobre 2002) décrit un ensemble pour le traitement thermique permettant de contrôler de manière automatique la température dans une région cible du tissu à traiter. L'ensemble comprend un générateur d'ultrasons, des moyens d'imagerie IRM pour mesurer et enregistrer la distribution spatiale de température dans la région cible et une unité de contrôle comprenant des moyens de traitement numérique point par point de la distribution spatiale de température. L'unité de contrôle commande le déplacement dans l'espace du générateur d'ultrasons en fonction de la distribution de température mesurée par les moyens d'imagerie de manière à ce que la température dans la région cible suive un profil de température de consigne.

Dans le cadre d'un traitement du sein, le positionnement des moyens générateurs d'énergie est délicat car le sein se trouve à proximité d'organes vitaux tels que la plèvre, les poumons ou le coeur. Or on souhaite éviter que la nécrose générée par l'élévation de température dans les tissus ne s'étende à ces organes vitaux.

Le problème résolu par l'invention est de proposer un dispositif de positionnement des moyens générateur d'énergie qui soit adapté au traitement du sein et qui minimise les risques d'endommagement des organes voisins.

Le document WO 03/059434 décrit un ensemble pour le traitement thermique du sein selon le préambule de la revendication 1.

L'invention résout ce problème grâce à un ensemble pour le traitement thermique du sein, conforme à la revendication 1.

Avec un tel ensemble de positionnement, le corps d'une patiente à traiter est maintenu dans une position dans laquelle le plan coronal du corps de la patiente s'étend sensiblement parallèlement à la direction de tir tandis que le sein de la patiente coupe cette direction de tir. L'énergie émise dans la direction de tir se propage uniquement à l'intérieur du sein et n'atteint jamais les organes vitaux.

Par plan coronal, on entend dans le cadre de la présente invention un plan passant par le centre de gravité du corps et qui divise le corps en une partie antérieure et une partie postérieure. Le plan coronal (ou plan frontal) s'étend perpendiculairement au plan sagittal (le plan sagittal étant le plan de symétrie du corps, contenant la colonne vertébrale, qui délimite une partie gauche et une partie droite du corps).

Les moyens de déplacement modifient la position des moyens générateurs d'énergie dans un plan de tir autour du sein pour ajuster la position du point focal des moyens générateurs d'énergie dans la zone cible à traiter et pour modifier la répartition de l'énergie au cours du temps en fonction par exemple de la distribution spatiale de température.

Dans une telle configuration, les risques d'accumulations d'énergie sur les organes à risques comme la plèvre, les poumons ou le coeur sont réduits puisque l'énergie ultrasonore se propage uniquement à l'intérieur du sein.

D'autres caractéristiques et avantages ressortiront encore de la description qui suit, laquelle est purement illustrative et non limitative et doit être lue en regard des figures annexées, parmi lesquelles :

- la figure 1 représente de manière schématique les différents éléments constitutifs d'un ensemble pour le traitement thermique du sein,
- la figure 2 représente le positionnement de moyens générateurs d'énergie sous la forme d'un transducteur à ultrasons focalisés par rapport au sein de la patiente,
- les figures 3, 4 et 5 sont des vues du transducteur à ultrasons focalisés respectivement en perspective, de dessus et de côté,
- la figure 6 représente de manière schématique le recouvrement dans l'espace entre deux tirs successifs du transducteur à ultrasons focalisés,
- les figures 7, 8 et 9 représentent des courbes d'intensité du champ acoustique généré par des transducteurs à ultrasons focalisés présentant des formes différentes,
- les figures 10, 11 et 12 représentent de manière schématique la répartition d'éléments générateurs d'ultrasons sur la surface active du transducteur respectivement en vue de face, en vue de côté et en vue de dessus,
- la figure 13 représente de manière schématique des moyens support adaptés pour maintenir le corps d'une patiente dans un ensemble de traitement thermique conforme à l'invention,
- la figure 14 représente de manière schématique un dispositif de positionnement des moyens générateurs d'énergie conforme à l'invention,

- la figure 15 représente de manière schématique des moyens générateurs d'énergie sous la forme d'un transducteur d'ultrasons focalisés muni de moyens d'interface destinés à favoriser la propagation des ultrasons dans le sein,
- la figure 16 représente de manière schématique un exemple d'architecture électronique d'un générateur multivoies destiné à alimenter un transducteur d'ultrasons focalisés,
- la figure 17 représente de manière schématique un circuit d'adaptation d'impédance entre une voie d'alimentation de générateur et un élément générateur d'ultrasons,
- la figure 18 représente de manière schématique le positionnement des moyens générateurs d'énergie ainsi que le positionnement de l'antenne de réception IRM à l'intérieur des moyens support prévus pour maintenir la patiente,
- la figure 19 représente plus précisément en vue de dessus les moyens support de la figure 13,
- la figure 20 représente de manière schématique en vue de dessus les moyens constitutifs du dispositif de positionnement de la figure 14, ces moyens de positionnement étant disposés sous les moyens support de la figure 19.

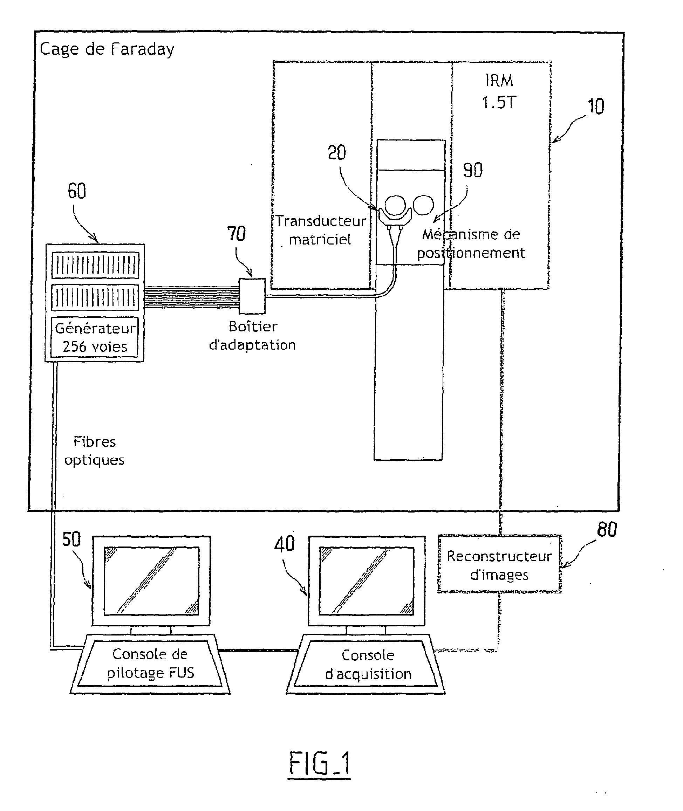

Sur la figure 1, l'ensemble de traitement thermique 1 représenté comprend un appareil d'imagerie IRM comportant un aimant 10. L'ensemble 1 comprend des moyens générateurs d'énergie sous la forme d'un transducteur ultrasons focalisés 20 et d'un générateur multivoie 60 destiné à alimenter le transducteur 20. Le générateur multivoie 60 (qui comprend au moins huit voies d'alimentation sinusoïdales) est relié au transducteur 20 par l'intermédiaire d'un dispositif électronique d'adaptation d'impédance 70.

L'ensemble 1 comprend également un reconstructeur d'images 80, une unité d'acquisition 40 incluant une unité centrale qui est apte à recevoir en entrée des données en provenance du reconstructeur d'images 80 et une unité de contrôle 50 qui est apte en fonction de données fournies par l'unité d'acquisition 40, à commander le générateur multivoie 60 et le système de déplacement 30 pour modifier la répartition de l'énergie émise par le transducteur 20 en fonction de la distribution de température mesurée par l'appareil d'imagerie IRM.

L'appareil d'imagerie IRM fournit simultanément une cartographie de la température tridimensionnelles de la zone d'intérêt et une cartographie anatomique avec une résolution spatiale de l'ordre du millimètre, une précision de l'ordre de 1 °C et une résolution temporelle de l'ordre de la seconde.

Le générateur multivoie comprend 256 voies, chaque voie étant destinée à l'alimentation d'un élément générateur d'ultrasons de la sonde 20. Les signaux générés sur chaque voie sont transmis via des câbles coaxiaux de 50Ω par le biais du dispositif électronique d'adaptation d'impédance 70 à la sonde 20. Le dispositif électronique d'adaptation d'impédance 70 joue également le rôle de filtre passe-bas pour permettre l'utilisation des ultrasons focalisés et de l'IRM simultanément et sans interférence comme décrit dans le document US 6 148 225 (publié le 14 novembre 2000).

Les mesures acquises à l'intérieur de l'aimant 10 sont converties en images par un reconstructeur d'images 80 et transmises à l'unité d'acquisition 40. L'unité d'acquisition 40 effectue une transformée de Fourier rapide et des filtrages de l'image acquise et affiche l'image ainsi traitée sur un écran. Le médecin peut visualiser l'image sur l'écran et localiser une tumeur à traiter.

Des cartographies de températures incluant la zone cible à chauffer sont effectuées par l'appareil d'imagerie IRM. Les données sont transférées en temps réel par une connexion réseau haut débit de l'unité d'acquisition 40 vers l'unité de contrôle 50 dédiée au pilotage des moyens générateurs d'ultrasons focalisés. L'unité de contrôle 50 inclut un programme adapté pour mettre en oeuvre un algorithme d'asservissement du générateur multivoie 60 et du système de déplacement 30. Cet algorithme est apte à calculer en fonction des cartes de températures fournies par l'appareil d'imagerie IRM les coordonnées et l'intensité des tirs d'ultrasons à effectuer en vue de générer une élévation thermique nécessaire à l'obtention d'une nécrose. L'algorithme d'asservissement commande le générateur multivoie 60 et le dispositif de déplacement 90 pour que la température dans la région cible suive un profil désiré.

Les signaux de commande émis par l'unité de contrôle 50 sont transmis au générateur multivoies 60, via des conducteurs à fibres optiques.

A la fin du traitement, des images IRM de diffusion ou en mode pondéré en T2 avec ou sans agent de contraste permettent d'effectuer un suivi du patient pour vérifier la taille de la nécrose engendrée ainsi que son évolution dans le temps.

LE TRANSDUSTEUR A ULTRASONS FOCALISES

L'inconvénient des sondes circulaires est la forme allongée du point focal le long de l'axe de tir. Lors du dépôt de grande quantité d'énergie pour effectuer de forte élévation de température ou le traitement de grand volume, la nécrose se propage alors vers le transducteur jusqu'à la surface de la peau.

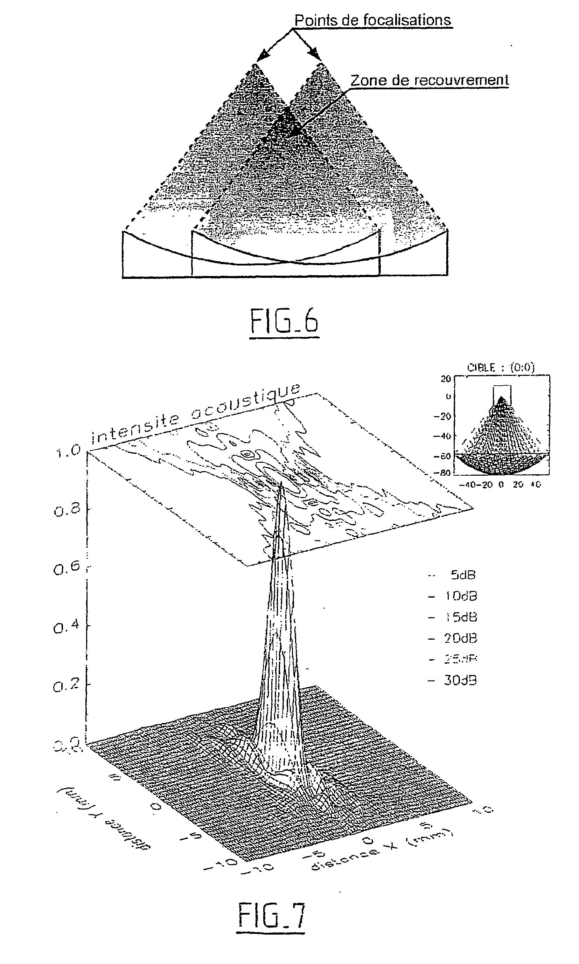

Comme on peut le voir sur la figure 6, lors de l'émission de plusieurs tirs consécutifs rapprochés, une partie de chaque faisceau se recouvre. Ainsi en faisant plusieurs tirs consécutifs sur toute la surface d'un disque on obtient un ellipsoïde dont le rapport des longueurs est égal au rapport des longueurs du point focal. Cet effet d'accumulation d'énergie le long de l'axe de propagation des ultrasons peut être dangereux lorsque la nécrose s'étend sur des organes voisins vitaux.

Le transducteur à ultrasons 20 quant à lui est particulièrement adapté pour traiter les tumeurs du sein.

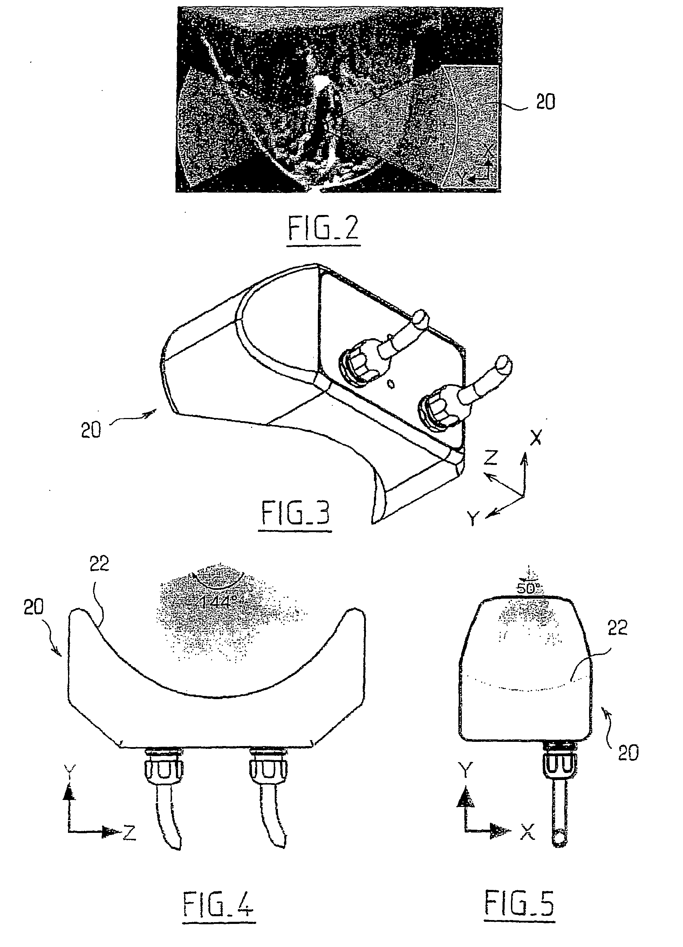

Sur le figure 2, le transducteur 20 est destiné à être positionné par rapport au sein de la patiente à traiter de sorte que la direction de tir des ultrasons s'étende sensiblement parallèlement au plan coronal de la patiente. Dans une telle configuration, les risques d'accumulations d'énergie sur les organes à risques comme la plèvre, les poumons ou le coeur sont réduits puisque l'énergie ultrasonore se propage uniquement à l'intérieur du sein.

Comme illustré sur les figures 3 à 5, le transducteur 20 présente une surface active d'émission d'ultrasons 22 de forme générale concave et allongée. Plus précisément, la surface active 22 du transducteur est une portion de sphère dont l'angle d'ouverture dans une première direction est supérieur à 90 degré tandis que l'angle d'ouverture dans une deuxième direction perpendiculaire à la première direction est inférieur à 90 degrés. La surface active 22 présentant un rayon de courbure de 8 cm et est allongée dans la direction du plan de tir. Ainsi le transducteur 20 permet de traiter des zones se trouvant jusqu'à 8cm de profondeur à l'intérieur du corps.

La figure 4 est une vue de dessus du transducteur, dans le plan de tir YZ. Dans ce plan, la surface active 22 présente un angle d'ouverture de 144 degrés. La figure 5 est une vue de côté du transducteur, dans un plan YX perpendiculaire au plan de tir YZ. Dans ce plan, la surface active présente un angle d'ouverture de 50 degrés. Pour obtenir une puissance suffisante la surface active est de $68cm^2$.

De plus en augmentant l'angle d'ouverture du transducteur, il est possible de diminuer la longueur du point focal. Sa disposition dans le plan coronal à la périphérie forme presque un demi-arc. Le demi-arc de cercle étant exclu pour éviter qu'une face de la sonde rayonne sur une autre face, ce qui pourrait provoquer son autodestruction. L'angle d'ouverture du transducteur dans le plan XY est de 144° (figure 4). Pour avoir un transducteur suffisamment compact c'est à dire avec une surface active de $68cm^2$, l'angle d'ouverture de la sonde dans le plan XZ est réduit à 50° (figure 5). Ceci permet de positionner le transducteur à proximité du corps comme le montre la figure 2.

Le fait d'augmenter l'angle d'ouverture du transducteur dans une direction permet de réduire la longueur du point focale. Ainsi avec un angle d'ouverture de 144° la longueur du point focal est proche de la longueur d'onde qui est ici de 1 mm.

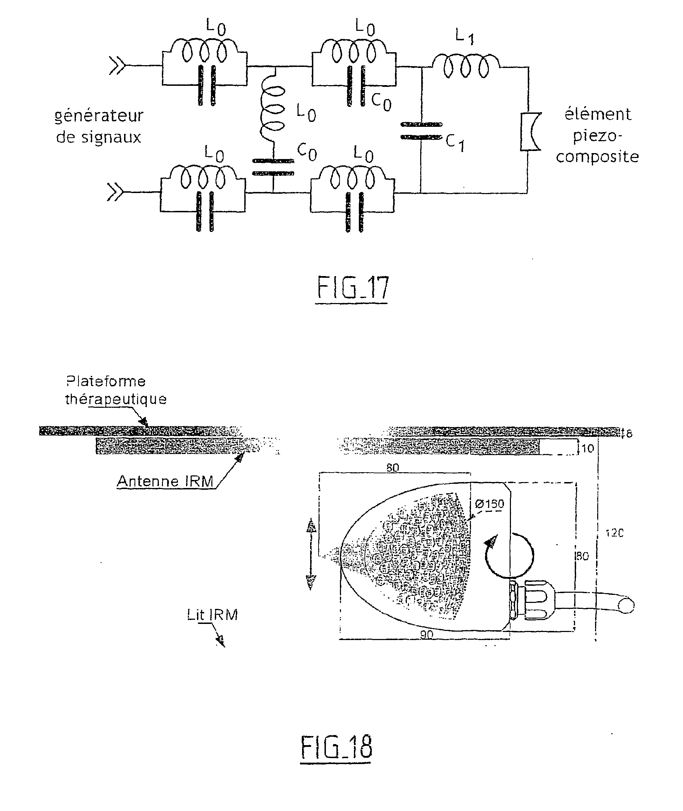

La forme allongée de ce transducteur permet en plus d'optimiser l'encombrement dans l'appareil d'imagerie IRM. En effet le diamètre des IRM est actuellement limité à 60 cm de rayon. Ceci empêche déjà de diagnostiquer par IRM certains patients atteints d'obésité. Avec une plateforme thérapeutique dans l'IRM, la place disponible pour le patient est encore plus réduite. Pour ces raisons, il est essentiel d'optimiser l'épaisseur de cette plateforme. Celle-ci dépend principalement de la taille du transducteur comme le montre la Figure 18. Le transducteur doit avoir une surface émettrice d'au moins $68 cm^2$ pour avoir une puissance suffisante et doit avoir une hauteur minimal dans la plateforme, il est préférable que celui-ci est un angle d'ouverture maximal avec un positionnement des éléments le plus dense possible. Toutefois l'angle d'ouverture du transducteur ne doit dépasser les 180° auquel cas le transducteur émettrait sur lui-même.

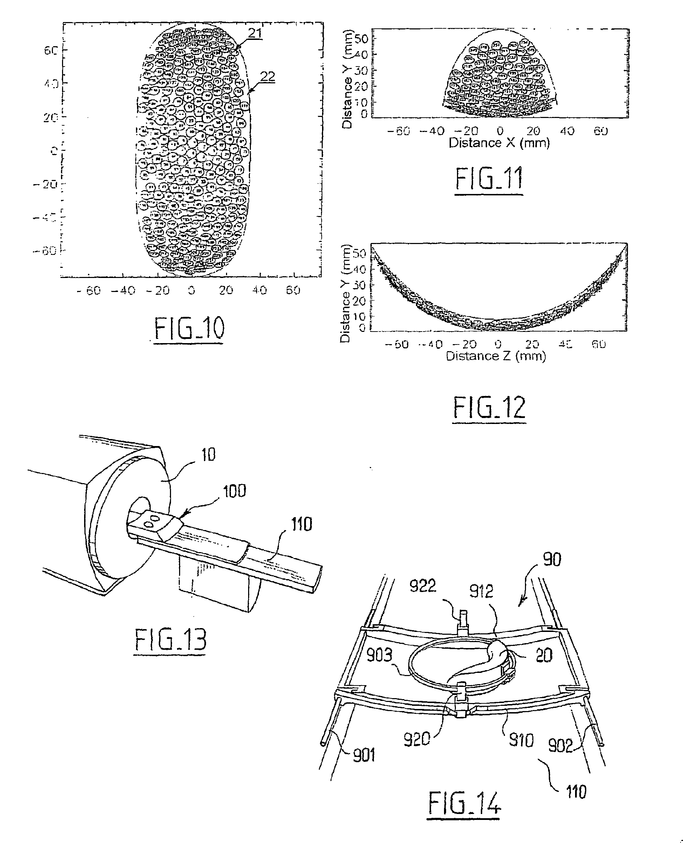

Comme illustré sur la figure 10, la surface active 22 du transducteur 20 est tapissée d'un réseau matriciel d'éléments générateurs d'ultrasons comportant 256 éléments circulaires générateurs aptes à émettre des ultrasons en direction d'un point focal P lorsque le transducteur 20 est alimenté par le générateur 60.

Lorsque le transducteur est alimenté par un générateur multivoie, via ses connexions, les éléments générateurs d'ultrasons 21 sont aptes à émettre à une fréquence d'environ 1,5 MHz. Le point focal P présente des dimensions de l'ordre de la longueur d'onde, c'est-à-dire environ 1 mm. La pression acoustique générée au point focal peut être ajustée en modifiant l'amplitude et le déphasage des signaux d'alimentation du générateur multivoies 60.

Le réseau matriciel formé par les éléments générateurs d'ultrasons (21) est compact et asymétrique. Le fait que le réseau matriciel soit compact signifie que chaque élément générateur 21 est contigu à au moins deux autres éléments pour minimiser la surface occupée par les éléments générateurs. Un élément générateur est considéré comme contigu à un autre élément générateur si ces éléments sont situés à une distance inférieure à 2 mm l'un de l'autre. Le fait que le réseau matriciel soit asymétrique signifie que les éléments générateurs 21 sont répartis de manière asymétrique sur la surface active 22.

Un algorithme itératif place les éléments un par un, selon le principe qui suit :

En connaissant les coordonnées des n premiers éléments, un programme calcule les positions des éléments contigus à au moins deux éléments parmi les n éléments pour minimiser la surface occupée par les n+1 élément. Sans considérer la distance minimum de 2 mm, les calculs peuvent être menés de la même façon que pour les réseaux matriciels épars en positionnant aléatoirement les éléments. Toutefois un espace entre les éléments est nécessaire pour éviter de créer des arcs électriques entre les connexions des éléments ou pour éviter les frictions mécaniques entres les éléments vibrant en opposition de phase. Cet espace inter-élément a été choisi égal à 0,4 mm dans le cas du transducteur ici décrit.

Parmi les configurations minimisant la surface occupée un programme permet de sélectionner celle qui présente le degré de symétrie le plus faible.

Selon un premier procédé pouvant être mis en oeuvre par ce programme, le degré de symétrie est évalué en cherchant pour chaque plan contenant l'axe Y le nombre d'éléments symétriques entre eux par rapport à l'un de ces plans. Certains éléments pouvant être quasiment symétriques l'un de l'autre sans l'être complètement, une fonction d'auto-corrélation permet de quantifier de façon continue le degré de symétrie plutôt que de façon discrète. La répartition des éléments retenue est alors celle pour laquelle chaque plan contenant l'axé Y retourne une auto-corrélation de symétrie minimum. Le fait de supprimer les axes de symétrie permet de diminuer les lobes de symétries qui sont le plus contraignants dans l'utilisation du réseau matriciel.

Selon un deuxième procédé itératif, plus rigoureux mais nécessitant un temps de calcul beaucoup plus long, pour chaque configuration minimisant la surface occupée, des simulations du champ acoustique sont effectuées comme illustré sur les figure s 7, 8 ou 9. Ce procédé permet de déterminer exactement la configuration minimisant les lobes secondaires générés par le transducteur.

Toutefois le premier procédé permet aussi de supprimer la répartition en couche sur le transducteur puisque lorsque deux éléments ont les mêmes coordonnées sur l'axe Y, ils sont exactement le symétrique l'un de l'autre pour un des plans passant par l'axe Y. De cette propriété découle une minimisation des lobes de réseaux.

Ces procédés conduisent à plusieurs répartitions différentes selon le choix de positionnement du premier élément. Pour lever cette indétermination, il est possible de comparer chaque répartition de 256 éléments pour choisir celle présentant les meilleures performances en termes de qualité de focalisation.

Le réseau matriciel ainsi obtenu présente les avantages de qualité de focalisation des réseaux semi-aléatoires épars comme décrit dans le document GRAVILLOV L. R. et al. « A theoretical assessment of the relative performance of spherical phased arrays for ultrasound surgery », IEEE transactions on ultrasonic, ferroelectrics and frequency control, janvier 2000, 47, 125-139, tout en étant compact. La compacité est un avantage pour un usage à l'intérieur d'un appareil d'imagerie IRM où la place disponible pour le transducteur est limitée par les dimensions de l'aimant.

Le réseau matriciel d'éléments générateurs 21 permet d'ajuster électroniquement la position du point focal P avec une précision de 7,5mm dans toutes les directions autour du point de focalisation naturel. De plus, la répartition compacte asymétrique des éléments 21 permet de conserver des lobes secondaires d'intensité inférieure de 8% à l'intensité au point de focalisation quelle que soit sa position dans la région décrite précédemment.

Les figures 7, 8 et 9 représentent des courbes d'intensité du champ acoustique généré par des transducteurs à ultrasons focalisés présentant des formes différentes. Ces figures permettent de comparer le champ acoustique généré par un transducteur de forme allongé avec le champ généré par un transducteur classique.

La figure 7 montre l'intensité du champ acoustique dans le plan XY sur une fenêtre de $20\times20mm^2$ autour du point focal P d'un transducteur matriciel circulaire représenté dans le coin supérieur gauche. Ce transducteur, comportant 256 éléments identiques, présente une distance focale de 80 mm et un rayon d'ouverture 55 m, ce qui conduit à un angle d'ouverture de 86 degrés. Le calcul de l'intensité du champ acoustique a été effectué pour une fréquence d'émission des éléments générateurs de 1,5 MHz dans un milieu de célérité de 1540 m/s. Ce calcul conduit à l'obtention d'un point focal de dimensions $0,76\times0,76\times3,5$ $mm^3$.

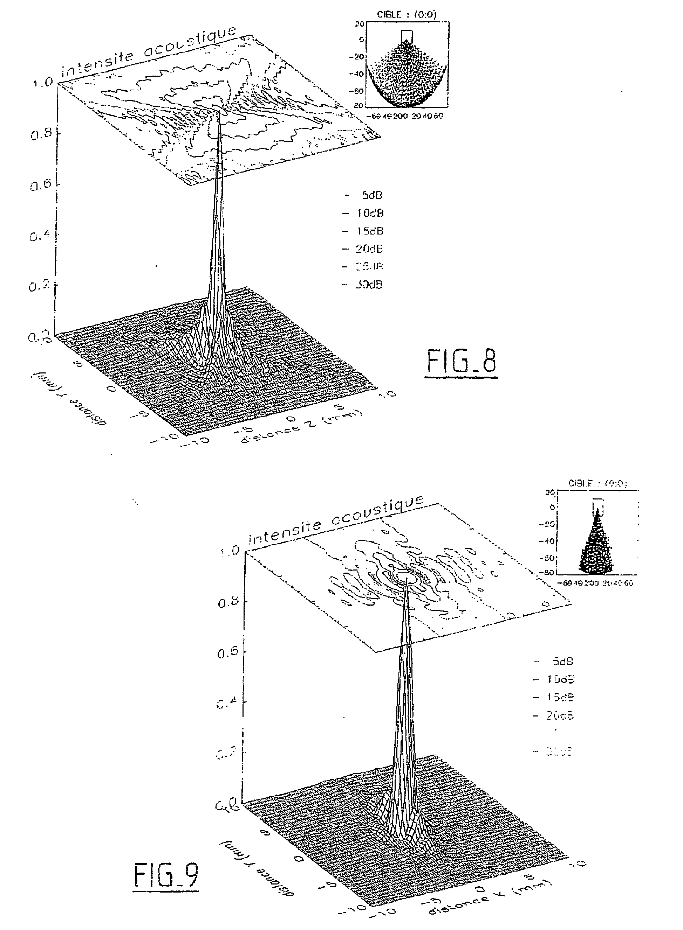

La figure 8 montre l'intensité du champ acoustique dans le plan YZ sur une fenêtre de 20x20 $mm^2$ autour du point focal P du transducteur décrit en relation avec la figure 3 et représenté dans le coin supérieur gauche. Ce transducteur présente également une distance focale de 80 mm et fonctionne à 1,5 MHz dans un milieu de célérité 1540m/s. Ce calcul conduit à l'obtention d'un point focal de dimensions 1,28x1,6 $mm^2$ dans le plan YZ.

La figure 9 montre l'intensité du champ acoustique dans le plan XY sur une fenêtre de $20\times20$ $mm^2$ autour du point focal P du transducteur décrit en relation avec la figure 3 et représenté dans le coin supérieur gauche. Avec les mêmes paramètres de calcul que ceux de la figure 7, on obtient un point focal de dimensions $0,48\times1,28\times1,6$ $mm^3$.

De plus pour pouvoir déplacer mécaniquement le centre de tir du transducteur vers le haut ou vers le bas (double flèche droite sur la figure 18), il est plus judicieux de faire pivoter le transducteur selon la flèche circulaire que de translater le transducteur. La translation nécessiterait un plus grand encombrement de la plateforme et ne permettrait pas d'atteindre la partie du sein prés de la cage thoracique. Pour avoir une liberté de rotation maximum du transducteur le long de l'axe bleu, celui-ci est découpé de sorte que sa vue de coté corresponde à une demi ellipse. En trois dimensions, il s'agit de l'intersection d'un cylindre elliptique avec une sphère. Une vue de coté triangulaire permettrait une plus grande rotation mais les coins arrondis son nécessaire à l'ergonomie et la rigidité du transducteur.

DISPOSITIF DE POSITIONNEMENT DU TRANDUCTEUR

Le pilotage électronique des signaux est rapide et précis. Toutefois, l'ajustement électronique de la position du point est limité et ne permet pas à lui seul d'atteindre toutes les zones du sein. Pour cette raison un centrage mécanique du transducteur sur le centre de la tumeur est nécessaire.

Sur l a figure 13, l'ensemble pour le traitement thermique du sein représenté, comprend des moyens support 100 adaptés pour maintenir le corps d'une patiente dans une position dans laquelle le plan coronal du corps de la patiente s'étend sensiblement parallèlement au plan de tir tandis que le sein de la patiente coupe le plan de tir.

Les moyens support sont formés d'une plateforme adaptée pour être positionnée sur le lit 110 du dispositif d'imagerie IRM 10 sur lequel la patiente s'allonge. Lorsque la plateforme 100 est positionnée sur le lit 110, elle s'étend sensiblement parallèlement au lit en ménageant un espace entre la plateforme et le lit. L'espace ménagé entre la plateforme et le lit est destiné à loger des moyens générateurs d'énergie comprenant le transducteur à ultrasons focalisés et un dispositif de positionnement du transducteur.

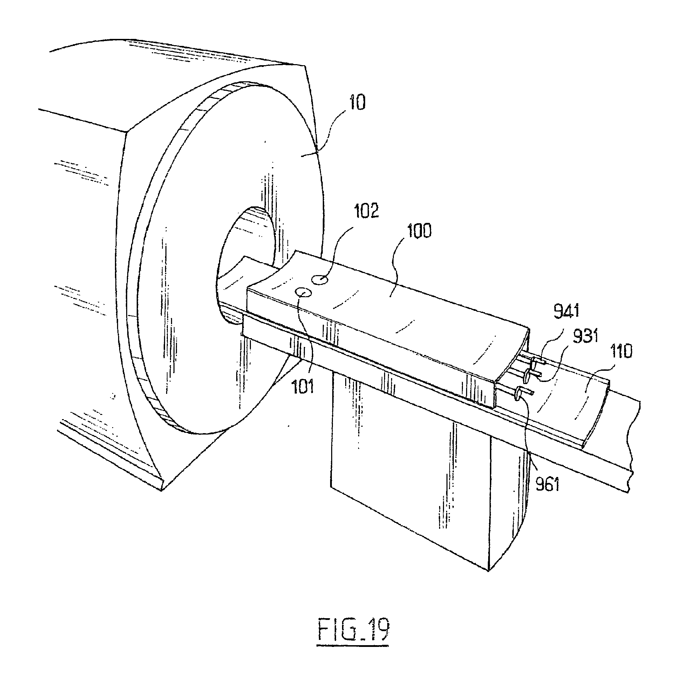

Comme on peut le voir plus précisément sur la figure 19, la plateforme 100 comprend deux orifices 101 et 102 agencés de sorte que lorsque la patiente s'étend à plat ventre sur le lit 110 de l'appareil d'imagerie IRM 10, les seins de la patiente sont introduits dans les orifices 101 et 102 en vue du traitement. La plateforme 100 ergonomique permet d'assurer le confort de la patiente tout en évitant que son poids ne repose sur le transducteur situé sous la plateforme.

Le dispositif de positionnement 90 est représenté sur la figure 14. Les moyens de déplacement sont adaptés pour maintenir les moyens générateur d'énergie 20 dans une position dans laquelle ces moyens générateurs 20 émettent de l'énergie dans une direction de tir parallèle au plan coronal de la patiente. Le dispositif de positionnement est également adapté pour modifier la position des moyens générateurs d'énergie 20 dans un plan de tir parallèle au plan coronal de la patiente, autour du sein de la patiente.

Le dispositif de déplacement comprend deux tiges 901 et 902 destinées à être positionnées le long des bords longitudinaux du lit 110 de l'appareil d'imagerie IRM sur lequel est positionnée la patiente et deux rails de guidage 910 et 912 venant en appui à chacune de leurs extrémités de manière coulissante sur les tiges 901 et 902 et s'étendant transversalement à la direction du lit 110. Chacun des deux rails 910 et 912 reçoit un montant référencé respectivement 920 et 922 s'étendant dans une direction générale perpendiculaire au lit 110 de l'appareil d'imagerie IRM et monté coulissant dans le rail 910 et 912 correspondant. Les deux montants 920 et 922 supportent un cadre 903 de forme générale annulaire sur lequel sont fixés les moyens générateurs d'énergie. Le cadre annulaire 903 délimite une zone centrale de tir dans laquelle est reçu le sein de la patiente.

Les moyens générateurs d'énergie comprennent un transducteur à ultrasons focalisés 20 présentant une surface active de forme générale sphérique. Le transducteur est fixé sur le cadre annulaire 903 de manière à ce que le plan de tir du transducteur 20 s'étende dans le plan du cadre annulaire 903 et le point focal naturel de la surface active sphérique du transducteur 20 soit positionné au centre du cadre 903.

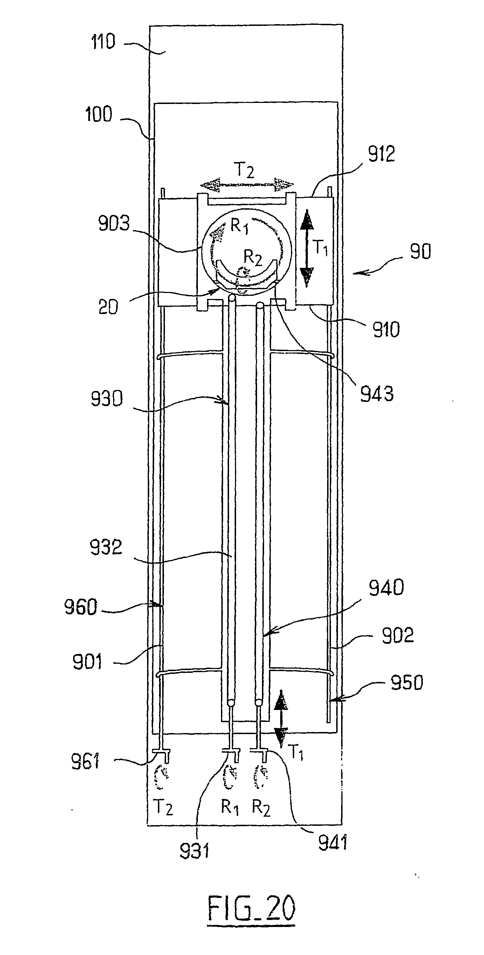

Comme on peut le voir plus précisément sur la figure 20, le dispositif de positionnement 90 comprend des premiers moyens de déplacement 930 adaptés pour modifier la position du cadre 903. Grâce à ces moyens de déplacement 930, le cadre 903 peut être entraîné en rotation autour de son centre (rotation $R_1$), dans le plan de tir, ce qui permet de modifier la position du transducteur 20 dans le plan de tir autour du sein de la patiente. A cet effet, le premier dispositif de positionnement 930 comprend un système mécanique à courroie et engrenages. La courroie de transmission 932 du premier dispositif de positionnement s'étend dans la longueur du lit et est commandée par une manivelle 931.

Par ailleurs, le dispositif de positionnement comprend des deuxièmes moyens de déplacement 940 adaptés pour modifier l'orientation du plan de tir, sous la forme d'un axe de rotation 943 s'étendant dans le plan du cadre 903. Les moyens générateurs d'énergie 20 sont montés rotatifs par rapport au cadre 903 sur l'axe 943. Les moyens générateurs d'énergie 20 peuvent donc légèrement basculer par rapport au plan coronal de la patiente (rotation $R_2$). Les deuxièmes moyens de déplacement 940 permettent un basculement du plan de tir avec un débattement compris entre +40 degrés et -40 degrés par rapport au cadre et donc par rapport au plan coronal de la patiente. Cette caractéristique offre une possibilité de réglage supplémentaire de la position du point focal P du transducteur à ultrasons.

Les moyens générateurs d'énergie 20 comprennent avantageusement un transducteur à ultrasons qui présente des contours arrondis, ce qui permet de modifier sa position et son orientation sans que le transducteur ne vienne buter contre le lit 110 ou les moyens support 100.

Par ailleurs, le dispositif de positionnement comprend des troisièmes moyens de déplacement en translation 950 comprenant les tiges 901 et 902 s'étendant le long des bords longitudinaux du lit 110. L'ensemble formé par le transducteur 20, le cadre annulaire 903, les montants et les rails 901, 912 peut être déplacé le long des tiges 901 et 902 selon une direction longitudinale du lit 110 (translation $T_1$). A cet effet, il suffit à un opérateur de saisir les manivelles 931 et 941 pour tirer ou pousser l'ensemble dans la direction longitudinale du lit 110.

Le dispositif de positionnement comprend des quatrièmes moyens de déplacement en translation 960 comprenant les rails de guidage 910 et 912. L'ensemble formé par le transducteur 20, le cadre annulaire 903 et les montants peuvent coulisser dans les rails 910 et 912 selon une direction transversale du lit 110 (translation $T_2$). Ainsi, l'ensemble peut être positionné dans la largeur du lit de l'appareil d'imagerie IRM. Le dispositif de déplacement 960 permet d'adapter la position du transducteur 20 en fonction de la position du sein de la patiente à traiter. A cet effet, les moyens de déplacement en translation 960 incluent la tige 901 à l'extrémité de laquelle est fixée la manivelle 961. Un opérateur peut commander la rotation de la tige 901 sur elle-même en actionnant la manivelle 961. La rotation de la tige 901 entraîne une courroie s'étendant dans une direction transversale au lit 110 (non représentée) et à laquelle est fixé l'ensemble formé par le transducteur 20, le cadre annulaire 903 et les montants. La courroie entraîne en translation l'ensemble le long des rails 910 et 912.

Le transducteur 20 peut ainsi être déplacé suivant 2 axes de translations et 2 axes de rotations grâce aux quatre moyens de déplacement 930, 940, 950 et 960 précédemment décrits. De cette façon il est possible de centrer le tir sur n'importe quelle partie à l'intérieur du sein tout en gardant une direction de tir sensiblement parallèle au plan coronal. Les moyens de déplacement 930, 940 et 960 sont commandés à distance par des manivelles 931, 941 et 961 se situant au pied du lit. Ces moyens de déplacement permettent donc d'ajuster la position du transducteur 20 sans modifier la position de la patiente sur le lit 110.

Le dispositif de positionnement et les moyens support offre nt une grande simplicité d'installation puisqu'il suffit de les poser directement sur le lit de l'appareil d'imagerie IRM.

En outre, le dispositif de positionnement décrit présente un encombrement réduit qui lui permet d'être reçu sous les moyens support et de conserver un maximum de place dans l'appareil d'imagerie IRM 10 pour la patiente.

MOYENS D'INTERFACE FAVORISANT LA PROPAGATION DES ULTRASONS

L'énergie émise par les moyens générateurs d'énergie à l'extérieur du corps de la patiente est pour une part transmise vers la zone à traiter à l'intérieur du corps de la patiente et pour une autre part perdue à cause des interfaces entre milieux de densités acoustiques différentes.

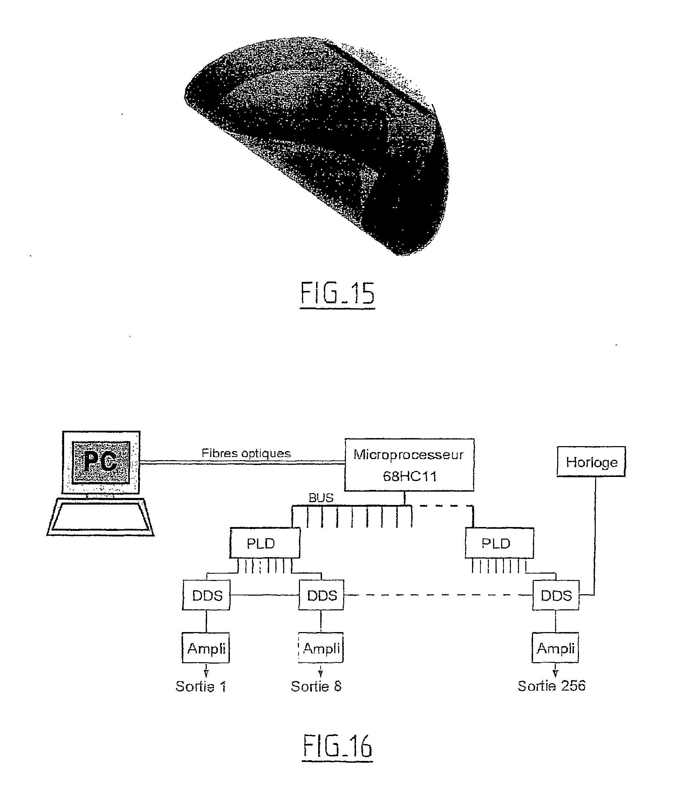

Une solution pour simplifier la transmission des ondes ultrasonores vers la zone cible à traiter est représentée à la figure 15.

La figure 15 représente un transducteur à ultrasons focalisés. La surface active du transducteur est recouverte d'une membrane déformable destinée à venir au contact de la peau de la patiente à traiter. L'espace créé entre la surface active du transducteur et la membrane est rempli d'eau. Cette caractéristique favorise la propagation des ondes ultrasonores générées par le transducteur vers l'intérieur du corps de la patiente. En effet, les ondes émises par les éléments générateurs d'ultrasons se propagent dans un milieu présentant une densité acoustique sensiblement constante.

Pour supprimer les effets de cavitation dans l'eau, celle-ci peut être préalablement dégazée avec une pompe à vide.

GENERATEUR D'ALIMENTATION MULTIVOIE

La figure 16 représente de manière schématique un exemple d'architecture électronique d'un générateur multivoie destiné à alimenter un transducteur d'ultrasons focalisés. Le générateur multivoie est commandé par une unité de contrôle de l'ensemble pour le traitement thermique. Le générateur multivoie 60 inclut au moins 256 voies d'alimentation sinusoïdales, aptes à être commandées indépendamment des unes des autres en amplitude et en phase et présentant un temps de commutation inférieur à 100 ms.

Sur la figure 16, l'unité de contrôle inclut une carte MOXA C104H permettant d'augmenter la vitesse du port série de 115200 Kb/s à 460800 Kb/s. L'unité de contrôle transmet au générateur multivoie des informations de fréquen ce, phases et amplitudes qui seront utilisées par le générateur pour générer des signaux de commande correspondants. Avec un tel système, les signaux de commande peu vent être complètement redéfinis toutes les 65 ms.

Le déplacement du point focal du transducteur à ultrasons focalisés est limité à un point toutes les 2 secondes avec une distance maximum de 4 mm entre chaque point, pour des pistons hydrauliques ayant une vitesse de 2 mm/s. En revanche ce générateur associé à un transducteur matriciel permet de chauffer en 15 points indépendants chaque seconde.

L'ensemble pour le traitement comprend un faisceau de fibres optiques permettant de connecter l'unité de contrôle située hors de la cage de Faraday entourant l'appareil d'imagerie IRM au générateur multivoie situé dans la cage de Faraday. Le faisceau de fibres optiques permet une transmission haut débit sur des grandes distances et constitue une excellente protection vis à vis des interférences électromagnétiques. Il permet de transmettre les informations générées par l'unité de contrôle à travers la cage de Faraday sans apporter de perturbations électromagnétiques en provenance de l'extérieur. La connexion entre l'unité de contrôle et le générateur est ainsi assurée sans interférer avec le fonctionnement de l'appareil d'imagerie IRM.

En outre, le générateur est conçu pour fonctionner à l'intérieur d'une salle IRM. Les cartes qu'il contient ont été choisies pour générer un faible rayonnement électromagnétique. Ainsi l'installation du générateur multivoie nécessite uniquement l'installation de deux fibres optiques.

Le générateur multivoie inclut un microprocesseur 68HC11. Le microprocesseur a pour fonction de distribuer les données aux circuits logiques programmables (PLD) sur les 32 cartes qui génèrent 8 sinusoïdes chacune. Les circuits logiques programmables vont ensuite transmettre des informations de phases et de fréquence aux générateurs de signaux numériques (DDS) et des informations de puissance aux amplificateurs. Les générateurs de signaux numériques (DDS) sont agencés en parallèles, et sont aptes en fonction des informations de phase et de fréquence qu'ils reçoivent à générer des signaux d'alimentation du réseau matriciel d'éléments générateurs d'ultrasons du transducteur. Les générateurs de signaux numériques assurent une commutation rapide et précise des signaux. Cette caractéristique représente une amélioration par rapport aux générateurs de signaux ultrasonores classiques à lignes à retard qui ne dépassent pas une précision en phase supérieure à $\pm 5°$. Les amplitudes des sinusoïdes sont ensuite ajustées par des amplificateurs à gain variable.

L'utilisation d'une horloge commune à tous les générateurs de signaux numériques assure une commutation synchronisée de tous les étages de sorties du générateur multivoie. Cette caractéristique permet d'éviter des états transitoires où les signaux ultrasons générés par le transducteur à ultrasons sont défocalisés. L'horloge commune améliore la sécurité du patient en maintenant la focalisation des signaux.

La définition des signaux par l'unité de contrôle se fait par la transmission au microprocesseur du générateur multivoie d'un fichier comportant les données selon l'ordre suivant :

- 4 octets optionnels codant la fréquence commune à tous les éléments générateurs d'ultrasons dont la valeur est :

$$F = 24 MHz \times \frac{Nb\ cod\acute{e}\ sur\ 32\ bits}{2^{32}}$$

La fréquence F est ainsi définie entre 0 et 24Mhz avec une précision de $\pm 3$mHz.

- 512 octets définissant sur 2 octets la phase $\phi$ respective de chaque signal de sorte que :

$$\phi = 360° \times \frac{Nb\ cod\acute{e}\ sur\ 16\ bits}{2^{12}}$$

Le codage sur 512 octets permet d'ajuster toutes les phases entre 0 et 360° avec une précision de $\pm 0,04°$.

- 256 octets définissant la puissance de sortie de chaque voie selon la relation :

$$P = 4W \times \frac{Nb\ cod\acute{e}\ sur\ 8\ bits}{2^{8}}$$

La puissance de sortie peut ainsi varier jusqu'à 4W avec une précision de $\pm 8$mW.

Le protocole de communication est au format X-MODEM en mode connecté half-duplex. Chaque paquet transmis de 128 octets est acquitté par le récepteur qui contrôle la réception par un caractère de contrôle cyclique redondant (CRC) codé sur 1 octet. De cette façon, l'unité de contrôle peut émettre un fichier définissant la fréquence, les phases ou les amplitudes toutes les 22ms.

L'architecture du générateur multivoie à 256 voies présente les avantages suivants :

- rapidité de redéfinition des signaux de contrôle,
- changement simultané des signaux,
- faible rayonnement électromagnétique,
- précision de la définition des sinusoïdes de sorties,
- un échange de donnés sécurisés,
- portabilité.

DISPOSITIF D'ADAPTATION D'IMPEDANCE

Sur chacune des 256 voies entre les sorties du générateur multivoie et les éléments générateurs d'énergie piézo -électriques est intercalé un dispositif électronique d'adaptation d'impédance représenté sur la figure 17. Les 256 circuits sont placés dans un boîtier disposé au pied de l'aimant pour être au plus près possible des moyens générateurs d'énergie sans toutefois gêner l'accès au centre de l'aimant.

Le rôle du dispositif d'adaptation d'impédance circuit est double.

Le dispositif d'adaptation d'impédance permet d'une part d'adapter l'impédance des éléments générateurs d'énergie piézo-électriques à $50\Omega$.

A cet effet, le dispositif représenté sur la figure 17 comprend des composants inductifs $L_1$ et capacitifs $C_1$

montés en filtre passe bas. On choisit les valeurs d'inductance et de capacité de la manière suivante :

$$C_1 = \frac{1}{50\Omega\omega}\sqrt{\frac{50\Omega}{Z_R}-1} \qquad L_1 = \frac{Z_R}{\omega}\sqrt{\frac{50\Omega}{Z_R}-1} - \frac{Z_I}{\omega}$$

où $Z_R$ et $Z_I$ sont la partie réelle et la partie imaginaire de l'impédance électrique d'un élément générateur d'énergie piézo-électrique. Avec de telles valeurs d'inductance et de capacité, on ramène l'impédance électrique vue par le générateur multivoie à 50Ω.

Les dispositifs électroniques d'adaptation permet tent un transfert optimal de l'énergie provenant du générateur par le biais de câble coaxiaux 50 Ω. La proximité des dispositifs électroniques d'adaptation d'impédance avec la sonde est nécessaire pour ne pas avoir une inductance $L_1$ de trop grande valeur ne fonctionnant pas en haute fréquence et engendrant trop de perte.

D'autre part, le dispositif d'adaptation d'impédance limite les interférences de l'appareil d'imagerie IRM (des gradients de champs) sur le générateur de signaux et également des signaux ultrasonores sur le signal RMN. A cet effet, les composants $L_1$ et $C_1$ réalisent un filtre passe-bas du $2^{ème}$ ordre. Cependant le comportement du filtre en haute fréquence est limité. Pour cette raison, un réseau de filtres bouchons résonnants à 64MHz $= \left(2\pi\sqrt{L_0 C_0}\right)^{-1}$ est intercalé entre la sortie du générateur multivoie et le filtre passe bas constitué par les composants inductifs $L_1$ et capacitifs $C_1$ pour atténuer les signaux à cette fréquence d'un facteur 200.

## Revendications

1.    Ensemble pour le traitement thermique du sein, comprenant

    - des moyens générateurs d'énergie (20) adaptés pour émettre de l'énergie dans une direction de tir incluse dans un plan de tir,
    - des moyens support adaptés pour maintenir le corps d'une patiente dans une position dans laquelle le plan coronal du corps de la patiente s'étend sensiblement parallèlement à la direction de tir tandis que le sein de la patiente coupe la direction de tir,
    - un dispositif de positionnement (90) incluant des moyens de déplacement (903, 930) adaptés pour modifier la position des moyens générateurs d'énergie (20) autour du sein de la patiente dans le plan de tir, le plan de tir s'étendant sensiblement parallèlement au plan coronal,

    caractérisé en ce que l'ensemble comprend un dispositif d'imagerie IRM (10) incluant un lit (110) et en ce que les moyens support sont formés d'une plateforme (100) positionnée sur le lit (110), les moyens générateurs d'énergie (20) et le dispositif de positionnement (90) étant logés dans un espace ménagé entre la plateforme (100) et le lit (110).

2.    Ensemble selon la revendication 1, dans lequel le dispositif de positionnement (90) comprend des moyens de déplacement (940, 950, 960) adaptés pour modifier la position du plan de tir.

3.    Ensemble selon la revendication 2, dans lequel les moyens de déplacement adaptés pour modifier la position du plan de tir comprennent des moyens de déplacement (940) adaptés pour modifier l'orientation du plan de tir.

4.    Ensemble selon la revendication 3, dans lequel les moyens de déplacement (940) adaptés pour modifier l'orientation du plan de tir permettent un basculement du plan de tir avec un débattement compris entre +40 degrés et -40 degrés.

5.    Ensemble selon l'une des revendications 1 à 4, dans lequel les moyens de déplacement adaptés pour modifier la position du plan de tir comprennent des moyens (950, 960) adaptés pour translater le plan de tir.

6.    Ensemble selon l'une des revendications qui précèdent, dans lequel les moyens de déplacement (903, 930) adaptés pour modifier la position des moyens générateurs d'énergie (20) dans le plan de tir comprennent un cadre de forme générale annulaire (903) sur lequel sont fixés les moyens générateurs d'énergie (20) et des moyens pour entraîner le cadre en rotation autour de son centre.

7. Ensemble selon l'une des revendications qui précèdent, dans lequel les moyens de déplacement (903, 930) adaptés pour modifier la position des moyens générateurs d'énergie (20) dans le plan de tir comprennent un système mécanique commandé par un moyen d'actionnement (931), le moyen d'actionnement (931) étant agencé à distance par rapport aux moyens générateurs d'énergie.

8. Ensemble selon la revendication 7, dans lequel le système mécanique inclut une courroie de transmission (932) actionnée par le moyen d'actionnement (931) et des engrenages.

9. Ensemble selon l'une des revendications 7 ou 8, dans lequel le moyen d'actionnement (931) comprend une manivelle.

10. Ensemble selon l'une des revendications 1 à 9, dans lequel les moyens générateurs d'énergie (20) comprennent un transducteur à ultrasons focalisés incluant une surface active (22) d'émission d'ultrasons, la surface active (22) présentant une forme générale concave et allongée.

11. Ensemble selon la revendication 10, dans lequel la surface active (22) du transducteur est une portion de sphère dont l'angle d'ouverture dans une première direction est supérieur à 90 degré tandis que l'angle d'ouverture dans une deuxième direction perpendiculaire à la première direction est inférieur à 90 degrés.

12. Ensemble selon l'une des revendications 10 ou 11, dans lequel la surface active (22) du transducteur est tapissée d'éléments générateurs d'ultrasons (21) formant un réseau matriciel.

13. Ensemble selon la revendication 12, dans lequel le réseau matriciel formé par les éléments générateurs d'ultrasons (21) est compact et asymétrique.

14. Ensemble selon l'une des revendications 1 à 13, comprenant des moyens d'alimentation (60) destinés à alimenter les moyens générateurs d'énergie et une unité de contrôle (50) apte à commander les moyens d'alimentation pour qu'ils génèrent des signaux d'alimentation, l'unité de contrôle (50) étant reliée aux moyens d'alimentation (60) par des éléments de transmission optiques aptes à transmettre des informations de commande de l'unité de contrôle d'alimentation vers les moyens d'alimentation (60).

15. Ensemble selon l'une des revendications 1 à 14, comprenant des moyens d'alimentation (60) destinés à alimenter les moyens générateurs d'énergie, les moyens d'alimentation incluant une pluralités de générateurs de signaux numériques (DDS) agencés en parallèles, aptes en fonction d'information de phase et de fréquence qu'ils reçoivent à générer des signaux d'alimentation d'un réseau matriciel d'éléments générateurs d'ultrasons des moyens générateurs d'ultrasons.

16. Ensemble selon l'une des revendications 1 à 15, comprenant des moyens d'alimentation (60) sous la forme d'un générateur électrique multivoie, incluant au moins 8 voies d'alimentation sinusoïdales, aptes à être commandées indépendamment des unes des autres en amplitude et en fréquence et présentant un temps de commutation inférieur à 100 ms.

**Claims**

1. An assembly for the thermal treatment of the breast, comprising:

   - power generator means (20) adapted for emitting power in a firing direction included in a plane of fire,
   - support means adapted for maintaining the body of a female patient in a position in which the coronal plane of the body of the female patient extends substantially in parallel with the firing direction, whereas the breast of the female patient intersects the firing direction,
   - a positioning device (90) including displacing means (903, 930) adapted for modifying the position of the power generator means (20) around the breast of the female patient in the plane of fire, the plane of fire extending substantially in parallel with the coronal plane,

   **characterised in that** the assembly comprises an imaging device MRI (10) including a bed (110) and **in that** the support means are formed by a platform (100) positioned on the bed (110), the power generator means (20) and the positioning device (90) being accommodated in a space provided between the platform (100) and the bed (110).

**2.** An assembly as claimed in claim 1, wherein the positioning device (90) comprises displacing means (940, 950, 960) adapted for modifying the position of the plane of fire.

**3.** Assembly as claimed in claim 2, wherein the displacing means adapted for modifying the position of the plane of fire comprise displacing means (940) adapted for modifying the orientation of the plane of fire.

**4.** Assembly as claimed in claim 3, wherein the displacing means (940) adapted for modifying the orientation of the plane of fire allow of tilting the plane of fire with an excursion comprised between +40 degrees and -40 degrees.

**5.** Assembly as claimed in any one of the claims 1 to 4, wherein the displacing means adapted for modifying the position of the plane of fire comprise means (950, 960) adapted for translating the plane of fire.

**6.** Assembly as claimed in any one of the preceding claims, wherein the displacing means (903, 930) adapted for modifying the position of the power generator means (20) in the plane of fire comprise a frame of general annular shape (903) to which are fixed the power generator means (20) and means for causing the frame to rotate around its centre.

**7.** Assembly as claimed in any one of the preceding claims, wherein the displacing means (903, 930) adapted for modifying the position of the power generator means (20) in the plane of fire comprise a mechanical system controlled by an actuating means (931), the actuating means (931) being arranged at a distance with respect to the power generator means.

**8.** Assembly as claimed in claim 7, wherein the mechanical system includes a transmission belt (932) actuated by the actuating means (931) and gear wheels.

**9.** Assembly as claimed in any one of the claims 7 or 8, wherein the actuating means (931) comprise a handle.

**10.** Assembly as claimed in any one of the claims 1 to 9, wherein the power generator means (20) comprise a focused ultrasound transducer including an active surface (22) for ultrasound transmissions, the active surface (22) presenting a general concave and elongated shape.

**11.** Assembly as claimed in claim 10, wherein the active surface (22) of the transducer is a spherical part of which the beam angle in a first direction is more than 90 degrees, whereas the beam angle in a second direction at right angles to the first direction is less than 90 degrees.

**12.** Assembly as claimed in any one of the claims 10 or 11, wherein the active surface (22) of the transducer is covered with ultrasound generator elements (21) forming a matrix network.

**13.** Assembly as claimed in claim 12, wherein the matrix network formed by the ultrasound generator elements (21) is compact and asymmetrical.

**14.** Assembly as claimed in any one of the claims 1 to 13, comprising power supply means (60) intended to supply power to the power generator means and a control unit (50) capable of controlling the power supply means so that they generate power supply signals, the control unit (50) being connected to the power supply means (60) by means of optical transmission elements capable of transmitting control data from the power supply control unit to the power supply means (60).

**15.** Assembly as claimed in any one of the claims 1 to 14, comprising power supply means (60) intended to supply power to the power generator means, the power supply means including a plurality of digital signal generators (DDS) connected in parallel, capable, in terms of phase and frequency information which they receive, of generating power supply signals for a matrix network of ultrasound generator elements of the ultrasound generator means.

**16.** Assembly as claimed in any one of the claims 1 to 15, comprising power supply means (60) in the form of a multichannel electrical generator, including at least 8 sinusoidal power supply channels, suitable for being amplitude and frequency-controlled independently of each other and presenting a switch time that is shorter than 100 ms.

**Patentansprüche**

1. Anordnung zur Wärmebehandlung der Brust, die Folgendes umfasst:

   - Energie erzeugende Mittel (20), die so ausgelegt sind, dass sie Energie in eine Zielrichtung abstrahlen, die Bestandteil einer Zielebene ist,
   - Auflagemittel, die so ausgelegt sind, dass sie den Körper einer Patienten in einer Position halten, in der sich die Frontalebene des Körpers der Patientin im Wesentlichen parallel zur Zielrichtung erstreckt, während die Brust der Patientin die Zielrichtung schneidet,
   - Positioniereinrichtung (90) mit Verstellmitteln (903, 930), die so ausgelegt sind, dass sie die Position der Energie erzeugenden Mittel (20) um die Brust der Patientin in der Zielebene verändern, wobei sich die Zielebene im Wesentlichen parallel zur Frontalebene erstreckt,

   **dadurch gekennzeichnet, dass** die Anordnung eine Magnetresonanz-Bildgebungseinheit (10) mit einem Tisch (110) umfasst und dass die Auflagemittel aus einer Plattform (100) bestehen, die auf dem Tisch (110) positioniert ist, wobei die Energie erzeugenden Mittel (20) und die Positioniereinrichtung (90) in einem zwischen der Plattform (100) und dem Tisch (110) ausgesparten Zwischenraum angeordnet sind.

2. Anordnung nach Anspruch 1, in der die Positioniereinrichtung (90) Verstellmittel (940, 950, 960) umfasst, die so ausgelegt sind, dass sie die Position der Zielebene verändern.

3. Anordnung nach Anspruch 2, in der die Verstellmittel, die so ausgelegt sind, dass sie die Position der Zielebene verändern, Verstellmittel (940) umfassen, die so ausgelegt sind, dass sie die Ausrichtung der Zielebene verändern.

4. Anordnung nach Anspruch 3, in der die Verstellmittel (940), die so ausgelegt sind, dass sie die Ausrichtung der Zielebene verändern, ein Kippen der Zielebene mit einer Verstellung zwischen +40° und -40° erlauben.

5. Anordnung nach einem der Ansprüche 1 bis 4, in der die Verstellmittel, die so ausgelegt sind, dass sie die Position der Zielebene verändern, Mittel (950, 960) umfassen, die so ausgelegt sind, dass sie die Zielebene verschieben.

6. Anordnung nach einem der vorhergehenden Ansprüche, in der die Verstellmittel (903, 930), die so ausgelegt sind, dass sie die Position der Energie erzeugenden Mittel (20) in der Zielebene verändern, einen im Allgemeinen ringförmigen Rahmen (903) umfassen, an dem die Energie erzeugenden Mittel (20) und Mittel zum Antrieb des Rahmens, der sich um seinen Mittelpunkt dreht, befestigt sind.

7. Anordnung nach einem der vorhergehenden Ansprüche, in der die Verfahrmittel (903, 930), die so ausgelegt sind, dass sie die Position der Energie erzeugenden Mittel (20) in der Zielebene verändern, ein mechanisches System umfassen, das von Betätigungsmitteln (931) gesteuert wird, wobei die Betätigungsmittel (931) mit einem Abstand zu den Energie erzeugenden Mitteln angeordnet sind.

8. Anordnung nach Anspruch 7, in der das mechanische System einen Antriebsriemen (932) umfasst, der von den Betätigungsmitteln (931) und Zahnrädern angetrieben wird.

9. Anordnung nach einem der Ansprüche 7 oder 8, in der die Betätigungsmittel (931) eine Kurbel umfassen.

10. Anordnung nach einem der Ansprüche 1 bis 9, in der die Energie erzeugenden Mittel (20) einen fokussierten Ultraschallwandler umfassen, der eine aktive Fläche (22) zur Ultraschallemission umfasst, wobei die aktive Fläche (22) eine im Allgemeinen konkave und längliche Form aufweist.

11. Anordnung nach Anspruch 10, in der die aktive Fläche (22) des Wandlers ein Kugelabschnitt ist, dessen Öffnungswinkel in einer ersten Richtung mehr als 90° beträgt, während der Öffnungswinkel in einer senkrecht zur ersten Richtung stehenden zweiten Richtung kleiner als 90° ist.

12. Anordnung nach einem der Ansprüche 10 oder 11, in der die aktive Fläche (22) des Wandlers mit Ultraschall erzeugenden Elementen (21) ausgekleidet ist, die ein Matrixnetz bilden.

13. Anordnung nach Anspruch 12, in der das aus den Ultraschall erzeugenden Elementen (21) gebildete Matrixnetz kompakt und asymmetrisch ist.

**14.** Anordnung nach einem der Ansprüche 1 bis 13, die Versorgungsmittel (60), die dazu bestimmt sind, die Energie erzeugenden Mittel zu versorgen, und eine Steuereinheit (50) umfasst, die die Versorgungsmittel derart steuern kann, dass sie Versorgungssignale erzeugen, wobei die Steuereinheit (50) mit den Versorgungsmitteln (60) durch optische Übertragungselemente verbunden ist, die Steuerinformationen von der Versorgungssteuereinheit zu den Versorgungsmitteln (60) übertragen können.

**15.** Anordnung nach einem der Ansprüche 1 bis 14, die Versorgungsmittel (60) umfassen, die dazu bestimmt sind, die Energie erzeugenden Mittel zu versorgen, wobei die Versorgungsmittel eine Vielzahl von parallel angeordneten Generatoren von digitalen Signalen (DDS) umfassen, die in Abhängigkeit von Phasen- und Frequenzinformationen, die sie empfangen, Signale zur Versorgung eines Matrixnetzes aus Ultraschall erzeugenden Elementen der Ultraschall erzeugenden Mittel erzeugen.

**16.** Anordnung nach einem der Ansprüche 1 bis 15, die Versorgungsmittel (60) in Form eines elektrischen Mehrkanal-Generators umfasst, die mindestens acht Versorgungskanäle für Sinussignale umfassen, die unabhängig voneinander hinsichtlich der Amplitude und der Frequenz gesteuert werden können und eine Schaltzeit von weniger als 100 ms aufweisen.

FIG.1

FIG.2

FIG.3

FIG.4

FIG.5

Points de focalisations

Zone de recouvrement

FIG_6

CIBLE : (0:0)

intensité acoustique

-- 5dB
— 10dB
— 15dB
— 20dB
— 25dB
— 30dB

distance Y (mm)

distance X (mm)

FIG_7

FIG_8

FIG_9

EP 1 755 742 B1

FIG.10

FIG.11

FIG.12

FIG.13

FIG.14

18

FIG.15

FIG.16

FIG_17

FIG_18

FIG.19

FIG_20

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2823678
- WO 03059434 A
- US 6148225 A

**Littérature non-brevet citée dans la description**

- **GRAVILLOV L. R. et al.** A theoretical assessment of the relative performance of spherical phased arrays for ultrasound surgery. *IEEE transactions on ultrasonic, ferroelectrics and frequency control,* Janvier 2000, vol. 47, 125-139